# EUROPEAN PATENT APPLICATION

(11) **EP 4 792 800 A1**
(43) Date of publication of application: **19.08.2026**
(21) Application number: 25315047.8
(22) Date of filing: 12.02.2025
(51) Int. Cl.: A61K 8/27, A61K 8/34, A61K 8/37, A61K 8/49, A61K 8/67, A61K 8/99, A61K 8/60, A61Q 19/08

(54) **COSMETIC OR DERMATOLOGIC OR PHARMACEUTICAL COMPOSITIONS FOR WELL-BEING AND/OR CELL REGENERATION**

(71) Applicant: TS-Biotech, 92088 Paris La Défense Cedex (FR); Shandong Tiansheng Biotechnology CO., LTD., Weifang City 262605 (CN)
(72) Inventor: Junjie, Jiang, Weifang City, 262605 (CN); Thibaut, Saguet, 45160 Olivet (FR); Yuhua, Jiang, Weifang City, 262605 (CN); Miao, Yu, Weifang City, 262605 (CN)
(74) Representative: Yes My Patent

(57) **Abstract**

The present invention relates to cosmetic or dermatologic or pharmaceutical compositions and non-therapeutical methods in the field of well-being and skin regeneration. The present invention further relates to the use of sialic acid and/or any derivatives thereof as an effective ingredient in a neuro-cosmetic or neuro-dermatologic composition for enhancing or promoting the synthesis of one or more of oxytocin, serotonin and beta-endorphin and/or for regenerating the sensory neuron neurite length in an ageing human cell.

## Description

The present invention relates to cosmetic or dermatologic or pharmaceutical compositions and non-therapeutical methods in the field of well-being and skin regeneration. The present invention further relates to the use of sialic acid and/or any derivatives thereof as an effective ingredient in a neuro-cosmetic or neuro-dermatologic composition for enhancing or promoting the synthesis of one or more of oxytocin, serotonin and beta-endorphin and/or for regenerating the sensory neuron neurite length in an ageing human cell.

The skin is the primary barrier of the human body which protects various organs from aggressions from pollution, UV and also changes such as fluctuating temperatures or humidity. It plays an important role in maintaining homeostasis *in vivo.* Additionally, it is known that endogenous factors, particularly emotional stress, significantly impact skin health. Exogeneous and endogenous factors can lead to premature aging, dehydration, loss of elasticity, and a compromised skin barrier. The disruption of skin homeostasis results in various dermatological concerns, including sensitivity, redness, irritation, and an overall fatigued complexion.

While traditional skincare formulations primarily focus on hydration, anti-aging, and barrier repair, it has been recently discovered that focusing on the hormone synthesis and peptides synthesis within the cells could address the deeper biochemical imbalances induced by stress, for example by regulating cortisol production. Thus, there is a need to provide new compositions for enhancing or promoting the synthesis of one or more of oxytocin, serotonin and beta-endorphin and/or for regenerating the sensory neuron neurite length in an ageing human cell in the field of well-being and skin regeneration.

According to the present invention, it has been found that sialic acid and/or any derivatives thereof permit to significantly enhance and promote the synthesis of one or more of oxytocin, serotonin and beta-endorphin, even it has been demonstrated that it promotes the synthesis of all three, namely oxytocin, serotonin and beta-endorphin, preferably by epidermal cells and/or neurons, more preferably by keratinocytes and/or neurons. Oxytocin, serotonin and beta-endorphin, each individually, plays a role in the moderation of the feeling of well-being, pleasure, serenity and so on such that it has been found that sialic acid and/or any derivatives thereof permits to induce a feeling of well-being, pleasure, and/or serenity in the treated person.

Therefore, the present invention relates to the use of sialic acid and/or any derivatives thereof as an effective ingredient in a neuro-cosmetic or neuro-dermatologic composition for enhancing or promoting the synthesis of one or more of oxytocin, serotonin and beta-endorphin and/or for regenerating the sensory neuron neurite length in an ageing human cell.

Preferably the use of sialic acid and/or any derivatives thereof as the effective ingredient in a neuro-cosmetic or neuro-dermatologic composition is for enhancing or promoting the synthesis of one or more of oxytocin, serotonin and beta-endorphin.

Preferably the synthesis of the one or more of oxytocin, serotonin and beta-endorphin is done by epidermal cells and/or neurons, more preferably by keratinocytes and/or neurons.

Preferably, as an alternative, the use of sialic acid arid/or any derivatives thereof as the effective ingredient in a neuro-cosmetic or neuro-dermatologic composition is for regenerating the sensory neuron neurite length in an ageing human cell.

### Well-being/Enhancement or promotion of the synthesis of one or more of oxytocin, serotonin and beta-endorphin:

The present invention further relates to a cosmetic or dermatologic or pharmaceutical composition comprising
an effective amount of a compound for use in enhancing or promoting the synthesis of one or more of oxytocin, serotonin and beta-endorphin;
wherein the compound for use in enhancing or promoting the synthesis of one or more of oxytocin, serotonin and beta-endorphin is sialic acid and/or any derivatives thereof.

It has been demonstrated that the composition of the present invention using sialic acid and/or any derivatives thereof permits to promote the synthesis of one or more of oxytocin, serotonin and beta-endorphin, even it has been demonstrated that it promotes the synthesis of oxytocin, serotonin and beta-endorphin, preferably by epidermal cells and/or neurons, more preferably by keratinocytes and/or neurons. Oxytocin is a hormone that promotes pleasure and attachment. Serotonin positively modulates mood and reduces pain. Beta-endorphin reduces stress and enhances well-being. Hence, using the composition of the present invention permits to induce a feeling of well-being, pleasure, and/or serenity in the treated person.

Preferably the compound for use in enhancing or promoting the synthesis of one or more of oxytocin, serotonin and beta-endorphin is sialic acid.

Preferably the effective amount of the compound for use in enhancing or promoting the synthesis of one or more of oxytocin, serotonin and beta-endorphin is of at least 0.01 wt.-%, based on the weight of the cosmetic or dermatologic or pharmaceutical composition.

Optionally the compound for use in enhancing or promoting the synthesis of one or more of oxytocin, serotonin and beta-endorphin being sialic acid and/or any derivatives thereof is encapsulated or vectorized.

Preferably the derivatives of sialic acid are one or more of O-methyl sialic acid, O-acetyl sialic acid, N-glycolylneuraminic acid, and silyl lactose.

### - Topical use

Preferably the cosmetic or dermatologic or pharmaceutical composition of the present invention is for topical use.

Preferably the effective amount of the compound for use in enhancing or promoting the synthesis of one or more of oxytocin, serotonin and beta-endorphin is in the range of from 0.01 to 10 weight-%, more preferably in the range of from 0.025 to 5 weight-%, more preferably in the range of from 0.05 to 1.5 weight-%, more preferably in the range of from 0.1 to 1.0 weight-%, based on the weight of the cosmetic or dermatologic or pharmaceutical composition.

Preferably the composition of the present invention is in the form of a cream, a serum, an oil-in-water emulsion, a water-in-oil emulsion, an oil, a gel, an aqueous solution, a vectorization system, an anhydrous stick, a foam, a lotion, a milk, or a camouflage product, more preferably in the form of a cream or a serum.

Preferably the cosmetic or dermatologic or pharmaceutical composition further comprises a cosmetic or dermatologic or pharmaceutical acceptable carrier, more preferably the acceptable carrier is selected from the group consisting of water, glycerin, glycol, propanediol, and mixtures of two or more thereof, more preferably selected from the group consisting of water, glycerin, and mixtures of two or more thereof.

Preferably the cosmetic or dermatologic or pharmaceutical composition further comprises one or more emulsifiers.

Preferably the content of emulsifier(s) in the cosmetic or dermatologic or pharmaceutical composition is in the range of from 1 to 20 wt.-%, more preferably in the range of from 1.5 to 10 wt.-%, more preferably in the range of from 1.7 to 8 wt.-%, based on the weight of the cosmetic or dermatologic or pharmaceutical composition.

Preferably the one or more emulsifiers are selected from the group consisting of a mixture of cetearyl olivate and sorbitan olivate; xanthan gum; ceteareth-6 olivate; cetearyl alcohol; ceteth-20; glyceryl stearate; steareth-20; a mixture of cetyl alcohol, glyceryl stearate, PEG-75 stearate, ceteth-20 and stearaeth-20; carbomer; a mixture of sodium acrylate and sodium acryloyldimethyl taurate copolymer; a mixture of beheneth-30 phosphate, cetostearyl alcohol, dicetyl phosphate and cetyl phosphate; and mixtures of two or more thereof; more preferably selected from the group consisting of a mixture of cetearyl olivate and sorbitan olivate; xanthan gum; ceteareth-6 olivate; and mixtures of two or more thereof.

Preferably the cosmetic or dermatologic or pharmaceutical composition further comprises one or more emollients.

Preferably the content of emollient(s) in the cosmetic or dermatologic or pharmaceutical composition is in the range of from 1 to 50 wt.-%, more preferably in the range of from 1.5 to 30 wt.-%, more preferably in the range of from 2 to 25 wt.-%, based on the weight of the cosmetic or dermatologic or pharmaceutical composition.

Preferably the one or more emollients are selected from the group consisting of isocetyl stearate; dicaprylyl carbonate; octyldodecanol; stearyl dimethicone; neopentyl glycol diethylhexanoate; cetyl ricinoleate; shorea robusta seed butter; dicaprylyl ether; cyclomethicone; a mixture of isodecane, hydrogenated tetradecenyl and methylpentadecene; pentaerythrityl tetralaurate; and mixtures of two or more thereof; more preferably selected from the group consisting of isocetyl stearate; dicaprylyl carbonate; octyldodecanol; stearyl dimethicone; neopentyl glycol diethylhexanoate; cetyl ricinoleate; shorea robusta seed butter; and mixtures of two or more thereof.

Preferably the cosmetic or dermatologic or pharmaceutical composition further comprises one or more of a pH regulator, a preservative, a thickening agent, a skin-care agent, and a perfuming agent.

In the present invention, any pH regulator can be used for the cosmetic or dermatologic or pharmaceutical composition as long as it provides its pH regulator/stabilizer properties and is cosmetically and dermatologically acceptable. Examples of pH regulator are sodium hydroxide, sodium bicarbonate, potassium hydroxide, triethanolamine, and aminomethyl propanol. Preferably in the present invention, sodium hydroxide is used as pH regulator.

In the present invention, any preservatives can be used for the cosmetic or dermatologic or pharmaceutical composition as long as it provides its preservative properties and is cosmetically and dermatologically acceptable. Examples of a preservative are selected from the group consisting of phenoxyethanol; propylparaben; methylparaben; butylparaben; sorbic acid; benzoic acid; benzyl alcohol; and mixtures of two or more thereof.

In the present invention, any thickening agents can be used for the cosmetic or dermatologic or pharmaceutical composition as long as it provides its thickening properties and is cosmetically and dermatologically acceptable. Examples of a thickening agent are a mixture of acrylates and C10-30 alkyl acrylate cross polymer; a mixture of polyacrylamide, C13-14 isoparaffin and laureth-7; xanthan gum; Sodium carboxymethyl starch; and/ or a mixture of Pullulan (and) Sorbitol (and) Trehalose (and) Acacia Senegal Gum.

In the present invention, any skin-care agents can be used for the cosmetic or dermatologic or pharmaceutical composition as long as it provides its skin caring properties and is cosmetically/dermatologically/pharmaceutically acceptable. Examples of a skin care agent are caprylyl methicone; vitamin C; and/or sodium hyaluronate.

In the present invention, any perfuming agents can be used for the cosmetic or dermatologic or pharmaceutical composition as long as it provides its flavoring properties and is cosmetically/dermatologically/pharmaceutically acceptable. Examples of a perfuming agent are tetradecane; essential oils, such as lavender, rose, jasmine; citrus extract.

Preferably the cosmetic or dermatologic or pharmaceutical composition is a serum. Preferably the composition comprises, more preferably consist of, sialic acid as the compound for use in enhancing or promoting the synthesis of one or more of oxytocin, serotonin and beta-endorphin; isocetyl stearate (as the emollient); a mixture of cetearyl olivate and sorbitan olivate, and xanthan gum (as the emulsifiers); phenoxyethanol (as the preservative); tetradecane (as the perfuming agent), sodium hydroxide (as the pH regulator); and water and glycerin (i.e. glycerin content between 2- 10 wt.-% based on the weight of the composition) as the cosmetic or dermatologic acceptable carrier.

Optionally, the cosmetic or dermatologic or pharmaceutical composition of the present invention further comprises one or more of pigments/dyes, antiseptics, antioxidants and UV-absorbing agent.

### - Oral Use

Preferably the cosmetic or dermatologic or pharmaceutical composition of the present invention is for oral use.

Preferably the effective amount of the compound for use in enhancing or promoting the synthesis of one or more of oxytocin, serotonin and beta-endorphin is in the range of from 1 to 100 weight-%, more preferably in the range of from 2 to 70 weight-%, more preferably in the range of from 4 to 55 weight-%, based on the weight of the cosmetic or dermatologic or pharmaceutical composition.

Preferably the cosmetic or dermatologic or pharmaceutical composition is in the form of a liquid, a capsule, a gummy, a powder, or a gel.

Preferably the cosmetic or dermatologic or pharmaceutical composition further comprises an antioxidant and/or anti-inflammatory component, more preferably said component being selected from the group consisting of zinc, OPCs, OPC derivatives, lutein, glutathione, selenium, vitamin C, blueberries, pomegranate, zinc bisglycinate, matcha tea, vitamin A, vitamin E, N-acetyl-cysteine, zeaxanthin, lycopene, turmeric, astaxanthin, and mixtures of two or more thereof, more preferably selected from the group consisting of zinc, OPCs, OPC derivatives, lutein, glutathione, selenium, vitamin C, blueberries, and mixtures of two or more thereof.

Preferably the OPC is a grape OPC.

Preferably the cosmetic or dermatologic or pharmaceutical composition further comprises one or more co-active ingredients, more preferably one or more of hyaluronic acid, collagen, superoxide dismutase (SOD), vitamins, polyphenols, peptides, saccharides, and lipides.

Preferably collagen is marine collagen.

Preferably hyaluronic acid is full spectrum hyaluronic acid.

Optionally the cosmetic or dermatologic or pharmaceutical composition further comprises a flavoring ingredient.

Optionally the cosmetic or dermatologic or pharmaceutical composition comprises one or more excipients. Any excipient known by the skilled person can be used as long as it is adapted for dietary purpose.
in the context of the present invention, the daily dosage of the compound for use in enhancing or promoting the synthesis of one or more of oxytocin, serotonin and beta-endorphin *(active ingredient)* preferably is in the range of from 10 mg/day to 1g/day, more preferably in the range of from 50 mg/day to 750 mg/day, more preferably in the range of from 100 mg/day to 500 mg/day.

Preferably the cosmetic or dermatologic or pharmaceutical composition being for oral use comprises sialic acid as the active ingredient, zinc, lutein, OPC (more preferably grapes OPC) and excipient(s). More preferably the content of sialic acid in this composition is in the range of from 3 to 7 weight-% based on the weight of the composition.

The present invention further relates to a non-therapeutical method for increasing the synthesis of one or more of oxytocin, serotonin and beta-endorphin, preferably by epidermal cells and/or neurons, more preferably by keratinocytes and/or neurons, in order to induce a feeling of well-being, pleasure, and/or serenity in a human person, comprising
applying a cosmetic or dermatologic or pharmaceutical composition for topical use according to the present invention to skin in need at least once a day, preferably twice a day; or
administering a cosmetic or dermatologic or pharmaceutical composition for oral use according to the present invention to a person in need once or twice a day.

Preferably the administration of the composition is repeated for a duration in the range of from 1 week to 6 months, more preferably in the range of from 2 weeks to 4 months.

Preferably the daily dosage of the compound for use in enhancing or promoting the synthesis of one or more of oxytocin, serotonin and beta-endorphin *(active ingredient)* is in the range of from 10 mg/day to 1g/day, more preferably in the range of from 50 mg/day to 750 mg/day, more preferably in the range of from 100 mg/day to 500 mg/day.

### Regeneration of sensory neuron neurite lenegth/regeneration of cells / anti-ageing:

The present invention further relates to a cosmetic or dermatologic or pharmaceutical composition comprising
an effective amount of a compound for use in regenerating the sensory neuron neurite length in an ageing human cell, and
wherein the compound for use in regenerating the sensory neuron neurite length in an ageing human cell is sialic acid and/or any derivatives thereof.

It has been demonstrated that by the present invention that the composition of the present invention using sialic acid and/or any derivatives thereof permits to regenerate the sensory neuron neurite length in an ageing human cell. Hence, using the composition of the present invention permits to regenerate the cells providing an anti-ageing effect in the treated person.

Preferably the compound for use in regenerating the sensory neuron neurite length in an ageing human cell is sialic acid.

Preferably the effective amount of the compound for use in regenerating the sensory neuron neurite length in an ageing human cell is of at least 0.01 wt.-%, based on the weight of the cosmetic or dermatologic or pharmaceutical composition.

Optionally the compound for use in regenerating the sensory neuron neurite length in an ageing human cell being sialic acid and/or any derivatives thereof is encapsulated or vectorized.

Preferably the derivatives of sialic acid are one or more of O-methyl sialic acid, O-acetyl sialic acid, N-glycolylneuraminic acid, and silyl lactose.

### - Topical use

Preferably the cosmetic or dermatologic or pharmaceutical composition of the present invention is for topical use.

Preferably the effective amount of the compound for use in regenerating the sensory neuron neurite length in an ageing human cell is in the range of from 0.01 to 10 weight-%, more preferably in the range of from 0.025 to 5 weight-%, more preferably in the range of from 0.05 to 1.5 weight-%, more preferably in the range of from 0.1 to 1.0 weight-%, based on the weight of the cosmetic or dermatologic or pharmaceutical composition.

Preferably the composition is in the form of a cream, a serum, an oil-in-water emulsion, a water-in-oil emulsion, an oil, a gel, an aqueous solution, a vectorization system, an anhydrous stick, a foam, a lotion, a milk, or a camouflage product, more preferably in the form of a cream or a serum.

Preferably the cosmetic or dermatologic or pharmaceutical composition further comprises a cosmetic or dermatologic or pharmaceutical acceptable carrier, more preferably the acceptable carrier is selected from the group consisting of water, glycerin, glycol, propanediol, and mixtures of two or more thereof, more preferably selected from the group consisting of water, glycerin, and mixtures of two or more thereof.

Preferably the cosmetic or dermatologic or pharmaceutical composition further comprises one or more emulsifiers.

Preferably the content of emulsifier(s) in the cosmetic or dermatologic or pharmaceutical composition is in the range of from 1 to 20 wt.-%, more preferably in the range of from 1.5 to 10 wt.-%, more preferably in the range of from 1.7 to 8 wt.-%, based on the weight of the cosmetic or dermatologic or pharmaceutical composition.

Preferably the one or more emulsifiers are selected from the group consisting of a mixture of cetearyl olivate and sorbitan olivate; xanthan gum; ceteareth-6 olivate; cetearyl alcohol; ceteth-20; glyceryl stearate; steareth-20; a mixture of cetyl alcohol, glyceryl stearate, PEG-75 stearate, ceteth-20 and stearaeth-20; carbomer; a mixture of sodium acrylate and sodium acryloyldimethyl taurate copolymer; a mixture of beheneth-30 phosphate, cetostearyl alcohol, dicetyl phosphate and cetyl phosphate; and mixtures of two or more thereof; more preferably selected from the group consisting of a mixture of cetearyl olivate and sorbitan olivate; xanthan gum; ceteareth-6 olivate; and mixtures of two or more thereof.

Preferably the cosmetic or dermatologic or pharmaceutical composition further comprises one or more emollients.

Preferably the content of emollient(s) in the cosmetic or dermatologic composition is in the range of from 1 to 50 wt.-%, more preferably in the range of from 1.5 to 30 wt.-%, more preferably in the range of from 2 to 25 wt.-%, based on the weight of the cosmetic or dermatologic or pharmaceutical composition.

Preferably the one or more emollients are selected from the group consisting of isocetyl stearate; dicaprylyl carbonate; octyldodecanol; stearyl dimethicone; neopentyl glycol diethylhexanoate; cetyl ricinoleate; shorea robusta seed butter; dicaprylyl ether; cyclomethicone; a mixture of isodecane, hydrogenated tetradecenyl and methylpentadecene; and mixtures of two or more thereof; more preferably selected from the group consisting of isocetyl stearate; dicaprylyl carbonate; octyldodecanol; stearyl dimethicone; neopentyl glycol diethylhexanoate; cetyl ricinoleate; shorea robusta seed butter; and mixtures of two or more thereof.

Preferably the cosmetic or dermatologic or pharmaceutical composition further comprises one or more of a pH regulator, a preservative, a thickening agent, a skin-care agent, and a perfuming agent.

In the present invention, any pH regulator can be used for the cosmetic or dermatologic or pharmaceutical composition as long as it provides its pH regulator/stabilizer properties and is cosmetically/dermatologically/pharmaceutically acceptable. Examples of pH regulator are sodium hydroxide, sodium bicarbonate, potassium hydroxide, triethanolamine, and aminomethyl propanol. Preferably in the present invention, sodium hydroxide is used as pH regulator.

In the present invention, any preservatives can be used for the cosmetic or dermatologic or pharmaceutical composition as long as it provides its preservative properties and is cosmetically/dermatologically/pharmaceutically acceptable. Examples of a preservative are selected from the group consisting of phenoxyethanol; propylparaben; methylparaben; butylparaben; sorbic acid; benzoic acid; benzyl alcohol; and mixtures of two or more thereof.

In the present invention, any thickening agents can be used for the cosmetic or dermatologic or pharmaceutical composition as long as it provides its thickening properties and is cosmetically/dermatologically/pharmaceutically acceptable. Examples of a thickening agent are a mixture of acrylates and C10-30 alkyl acrylate cross polymer; a mixture of polyacrylamide, C13-14 isoparaffin and laureth-7; xanthan gum; Sodium carboxymethyl starch; and/ or a mixture of Pullulan (and) Sorbitol (and) Trehalose (and) Acacia Senegal Gum.

In the present invention, any skin-care agent can be used for the cosmetic or dermatologic or pharmaceutical composition as long as it provides its skin caring properties and is cosmetically/dermatologically/pharmaceutically acceptable. Examples of a skin care agent are caprylyl methicone; vitamin C; and/or sodium hyaluronate.

In the present invention, any perfuming agent can be used for the cosmetic or dermatologic or pharmaceutical composition as long as it provides its flavoring properties and is cosmetically/dermatologically/pharmaceutically acceptable. Examples of a perfuming agent are tetradecane; essential oils, such as lavender, rose, jasmine; citrus extract.

Preferably the cosmetic or dermatologic or pharmaceutical composition is a cream. Preferably the composition comprises, more preferably consist of, sialic acid as the compound for use in regenerating the sensory neuron neurite length in an ageing human cell ; octyldodecanol, dicaprylyl carbonate, stearyl dimethicone, neo pentyl glycol diethylhexanoate, cetyl ricinoleate and shorea robusta seed butter (as the emollients); a mixture of cetearyl olivate and sorbitan olivate, and ceteareth-6 olivate (as the emulsifiers); a mixture of acrylates and C10-30 alkyl acrylate cross polymer (as the thickening agent); phenoxyethanol (as the preservative); caprylyl methicone (as the skin-care agent), sodium hydroxide (as the pH regulator) and water as the cosmetic or dermatologic acceptable carrier.

Optionally, the cosmetic or dermatologic or pharmaceutical composition of the present invention further comprises one or more of pigments/dyes, antiseptics, antioxidants and UV-absorbing agent.

### - Oral use

Preferably the cosmetic or dermatologic or pharmaceutical composition of the present invention is for oral use.

Preferably the effective amount of the compound for use in regenerating the sensory neuron neurite length in an ageing human cell is in the range of from 1 to 100 weight-%, more preferably in the range of from 1 to 95 weight-%, more preferably in the range of from 2 to 70 weight-%, more preferably in the range of from 4 to 55 weight-%, based on the weight of the cosmetic or dermatologic or pharmaceutical composition.

Preferably the cosmetic or dermatologic or pharmaceutical composition is in the form of a liquid, a capsule, a gummy, a powder, or a gel.

Preferably the composition further comprises an antioxidant and/or anti-inflammatory component, more preferably said component being selected from the group consisting of zinc, OPCs, OPC derivatives, lutein, glutathione, selenium, vitamin C, blueberries, pomegranate, zinc bisglycinate, matcha tea, vitamin A, vitamin E, N-acetyl-cysteine, zeaxanthin, lycopene, turmeric, astaxanthin, and mixtures of two or more thereof, more preferably selected from the group consisting of zinc, OPCs, OPC derivatives, lutein, glutathione, selenium, vitamin C, blueberries, and mixtures of two or more thereof.

Preferably the OPC is a grape OPC.

Preferably the composition further comprises one or more co-active ingredients, more preferably one or more of hyaluronic acid, collagen, superoxide dismutase (SOD), vitamins, polyphenols, peptides, saccharides, and lipides.

Preferably collagen is marine collagen.

Preferably hyaluronic acid is full spectrum hyaluronic acid.

Optionally the cosmetic or dermatologic or pharmaceutical composition further comprises a flavoring ingredient.

Optionally the cosmetic or dermatologic composition comprises one or more excipients. Any excipient known by the skilled person can be used as long as it is adapted for dietary purpose.

In the context of the present invention, preferably the daily dosage of the compound for use in regenerating the sensory neuron neurite length in an ageing human cell *(active ingredient)* is in the range of from 10 mg/day to 1g/day, more preferably in the range of from 50 mg/day to 750 mg/day, more preferably in the range of from 100 mg/day to 500 mg/day.

Preferably the cosmetic or dermatologic or pharmaceutical composition being for oral use comprises sialic acid as the active ingredient, glutathione, superoxide dismutase (SOD), selenium and excipient(s). More preferably the content of sialic acid in this composition is in the range of from 45 to 55 weight-% based on the weight of the composition.

Preferably the cosmetic or dermatologic or pharmaceutical composition being for oral use comprises sialic acid as the active ingredient, hyaluronic acid (more preferably full spectrum hyaluronic acid), collagen (more preferably marine collagen), vitamin C (more preferably liposomal vitamin C), blueberries powder and a flavoring agent, such as blueberries flavoring. More preferably the content of sialic acid in this composition is in the range of from 5 to 15 weight-% based on the weight of the composition.

The present invention further relates to a non-therapeutical method for regenerating the sensory neuron neurite length in an ageing human cell, comprising
- applying a cosmetic or dermatologic or pharmaceutical composition for topical use according to the present invention to a skin in need at least once a day, preferably twice a day; or
administering a cosmetic or dermatologic or pharmaceutical composition for oral use according to the present invention to a person in need once or twice a day.

Preferably the administration of the composition is repeated for a duration in the range of from 1 week to 6 months, more preferably in the range of from 2 weeks to 4 months.

Preferably the daily dosage of the compound for use in regenerating the sensory neuron neurite length in an ageing human cell (active ingredient) is in the range of from 10 mg/day to 1g/day, more preferably in the range of from 50 mg/day to 750 mg/day, more preferably in the range of from 100 mg/day to 500 mg/day.

The present invention further relates to sialic acid and/or any derivatives thereof for use in regenerating the sensory neuron neurite length in an ageing human cell, preferably an ageing human epidermal cell.

The present invention relates to the use of sialic acid as the active ingredient in a cosmetic or dermatologic composition for anti-ageing and/or skin regeneration.

The present invention relates to the use of sialic acid and/or any derivatives thereof for enhancing or promoting the synthesis of one or more of oxytocin, serotonin and beta-endorphin, preferably by epidermal cells and/or neurons, more preferably by keratinocytes and /or neurons.

The present invention is further illustrated by the following set of embodiments and combinations of embodiments resulting from the dependencies and back references as indicated. In particular, it should be noted that in each case where a range of embodiments is mentioned, for example in the context of a term such as "The composition of any one of embodiments 1 to 4", each embodiment in that range is intended to be explicitly disclosed to those skilled in the art, i.e., the wording of that term should be understood by those skilled in the art to be synonymous with "The composition of any one of embodiments 1, 2, 3 and 4". Furthermore, it is explicitly noted that the following set of embodiments represents a suitably structured part of the general description directed to preferred aspects of the present invention and, therefore, suitably supports, but does not represent, the claims of the present invention.

According to embodiment 1 of the present invention, a use of sialic acid and/or any derivatives thereof as an effective ingredient in a neuro-cosmetic or neuro-dermatologic composition for enhancing or promoting the synthesis of one or more of oxytocin, serotonin and beta-endorphin and/or for regenerating the sensory neuron neurite length in an ageing human cell is defined.

Embodiment 2 The use of embodiment 1, wherein the sialic acid and/or any derivatives thereof is the effective ingredient in a neuro-cosmetic or neuro-dermatologic composition for enhancing or promoting the synthesis of one or more of oxytocin, serotonin and beta-endorphin.

Embodiment 3 The use of embodiment 1, wherein the sialic acid and/or any derivatives thereof is the effective ingredient in a neuro-cosmetic or neuro-dermatologic composition for regenerating the sensory neuron neurite length in an ageing human cell.

Embodiment 4 Cosmetic or dermatologic or pharmaceutical composition comprising
an effective amount of a compound for use in enhancing or promoting the synthesis of one or more of oxytocin, serotonin and beta-endorphin;
wherein the compound for use in enhancing or promoting the synthesis of one or more of oxytocin, serotonin and beta-endorphin is sialic acid and/or any derivatives thereof.

Embodiment 5 The cosmetic or dermatologic or pharmaceutical composition of embodiment 4, wherein the compound for use in enhancing or promoting the synthesis of one or more of oxytocin, serotonin and beta-endorphin is sialic acid.

Embodiment 6 The cosmetic or dermatologic or pharmaceutical composition of embodiment 4 or 5, wherein the effective amount of the compound for use in enhancing or promoting the synthesis of one or more of oxytocin, serotonin and beta-endorphin is of at least 0.01 wt.-%, based on the weight of the cosmetic or dermatologic or pharmaceutical composition.

Embodiment 7 The cosmetic or dermatologic or pharmaceutical composition of any one of embodiments 4 to 6, being for topical use.

Embodiment 8 The cosmetic or dermatologic or pharmaceutical composition of embodiment 7, wherein the effective amount of the compound for use in enhancing or promoting the synthesis of one or more of oxytocin, serotonin and beta-endorphin is in the range of from 0.01 to 10 weight-%, preferably in the range of from 0.025 to 5 weight-%, more preferably in the range of from 0.05 to 1.5 weight-%, more preferably in the range of from 0.1 to 1.0 weight-%, based on the weight of the cosmetic or dermatologic or pharmaceutical composition.

Embodiment 9 The cosmetic or dermatologic or pharmaceutical composition of embodiment 7 or 8, being in the form of a cream, a serum, an oil-in-water emulsion, a water-in-oil emulsion, an oil, a gel, an aqueous solution, a vectorization system, an anhydrous stick, a foam, a lotion, a milk, or a camouflage product, preferably in the form of a cream or a serum.

Embodiment 10 The cosmetic or dermatologic or pharmaceutical composition of any one of embodiments 4 to 9, further comprising a cosmetic or dermatologic or pharmaceutical acceptable carrier, preferably the acceptable carrier is selected from the group consisting of water, glycerin, glycol, propanediol, and mixtures of two or more thereof, more preferably selected from the group consisting of water, glycerin, and mixtures of two or more thereof.

Embodiment 11 The cosmetic or dermatologic or pharmaceutical composition of any one of embodiments 4 to 10, further comprising one or more emulsifiers.

Embodiment 12 The cosmetic or dermatologic or pharmaceutical composition of embodiment 11, wherein the one or more emulsifiers are selected from the group consisting of a mixture of cetearyl olivate and sorbitan olivate; xanthan gum; ceteareth-6 olivate; cetearyl alcohol; ceteth-20; glyceryl stearate; steareth-20; a mixture of cetyl alcohol, glyceryl stearate, PEG-75 stearate, ceteth-20 and stearaeth-20; carbomer; a mixture of sodium acrylate and sodium acryloyldimethyl taurate copolymer; a mixture of beheneth-30 phosphate, cetostearyl alcohol, dicetyl phosphate and cetyl phosphate; and mixtures of two or more thereof, preferably selected from the group consisting of a mixture of cetearyl olivate and sorbitan olivate; xanthan gum; ceteareth-6 olivate; and mixtures of two or more thereof.

Embodiment 13 The cosmetic or dermatologic or pharmaceutical composition of any one of embodiments 4 to 12, further comprising one or more emollients.

Embodiment 14 The cosmetic or dermatologic or pharmaceutical composition of embodiments 13, wherein the one or more emollients are selected from the group consisting of isocetyl stearate; dicaprylyl carbonate; octyldodecanol; stearyl dimethicone; neopentyl glycol diethylhexanoate; cetyl ricinoleate; shorea robusta seed butter; dicaprylyl ether; cyclomethicone; a mixture of isodecane, hydrogenated tetradecenyl and methylpentadecene; pentaerythrityl tetralaurate; and mixtures of two or more thereof, preferably selected from the group consisting of isocetyl stearate; dicaprylyl carbonate; octyldodecanol; stearyl dimethicone; neopentyl glycol diethylhexanoate; cetyl ricinoleate; shorea robusta seed butter; and mixtures of two or more thereof.

Embodiment 15 The cosmetic or dermatologic or pharmaceutical composition of any one of embodiments 4 to 14, further comprising one or more of a pH regulator, a preservative, a thickening agent, a skin-care agent, and a perfuming agent.

Embodiment 16 The cosmetic or dermatologic or pharmaceutical composition of any one of embodiments 4 to 6, being for oral use.

Embodiment 17 The cosmetic or dermatologic or pharmaceutical composition of embodiment 16, wherein the effective amount of the compound for use in enhancing or promoting the synthesis of one or more of oxytocin, serotonin and beta-endorphin is in the range of from 1 to 100 weight-%, preferably in the range of from 2 to 70 weight-%, more preferably in the range of from 4 to 55 weight-%, based on the weight of the cosmetic or dermatologic or pharmaceutical composition.

Embodiment 18 The cosmetic or dermatologic or pharmaceutical composition of embodiment 16 or 17, being in the form of a liquid, a capsule, a gummy, a powder, or a gel.

Embodiment 19 The cosmetic or dermatologic or pharmaceutical composition of any one of embodiments 16 to 18, further comprising an antioxidant and/or anti-inflammatory component, preferably said component being selected from the group consisting of zinc, OPCs, OPC derivatives, lutein, glutathione, selenium, vitamin C, blueberries, pomegranate, zinc bisglycinate, matcha tea, vitamin A, vitamin E, N-acetyl-cysteine, zeaxanthin, lycopene, turmeric, astaxanthin, and mixtures of two or more thereof, more preferably selected from the group consisting of zinc, OPCs, OPC derivatives, lutein, glutathione, selenium, vitamin C, blueberries, and mixtures of two or more thereof.

Embodiment 20 The cosmetic or dermatologic or pharmaceutical composition of any one of embodiments 16 to 19, further comprising one or more co-active ingredients, preferably one or more of hyaluronic acid, collagen, superoxide dismutase (SOD), vitamins, polyphenols, peptides, saccharides, and lipides.

Embodiment 21 The cosmetic or dermatologic or pharmaceutical composition of any one of embodiments 16 to 20, further comprising a flavoring ingredient.

Embodiment 22 The cosmetic or dermatologic or pharmaceutical composition of any one of embodiments 16 to 21, wherein the daily dosage of the compound for use in enhancing or promoting the synthesis of one or more of oxytocin, serotonin and beta-endorphin *(active ingredient)* is in the range of from 10 mg/day to 1g/day, preferably in the range of from 50 mg/day to 750 mg/day, more preferably in the range of from 100 mg/day to 500 mg/day.

Embodiment 23 A non-therapeutical method for increasing the synthesis of one or more of oxytocin, serotonin and beta-endorphin, preferably by epidermal cells and/or neurons, more preferably by keratinocytes and/or neurons, in order to induce a feeling of well-being, pleasure, and/or serenity in a human person, comprising
applying a cosmetic or dermatologic or pharmaceutical composition for topical use according to any one of embodiments 4 to 15 to skin in need at least once a day, preferably twice a day; or
administering a cosmetic or dermatologic or pharmaceutical composition for oral use according to any one of embodiments 4 to 6 and 16 to 22 to a person in need once or twice a day.

Embodiment 24 The non-therapeutical method of embodiment 23, wherein the administration of the composition is repeated for a duration in the range of from 1 week to 6 months, preferably in the range of from 2 weeks to 4 months.

Embodiment 25 The non-therapeutical method of embodiment 23 or 24, wherein the daily dosage of the compound for use in enhancing or promoting the synthesis of one or more of oxytocin, serotonin and beta-endorphin *(active ingredient)* is in the range of from 10 mg/day to 1g/day, preferably in the range of from 50 mg/day to 750 mg/day, more preferably in the range of from 100 mg/day to 500 mg/day.

Embodiment 26 Cosmetic or dermatologic or pharmaceutical composition comprising
an effective amount of a compound for use in regenerating the sensory neuron neurite length in an ageing human cell, and
wherein the compound for use in regenerating the sensory neuron neurite length in an ageing human cell is sialic acid and/or any derivatives thereof.

Embodiment 27 The cosmetic or dermatologic or pharmaceutical composition of claim 26, wherein the compound for use in regenerating the sensory neuron neurite length in an ageing human cell is sialic acid.

Embodiment 28 The cosmetic or dermatologic or pharmaceutical composition of embodiment 26 or 27, being for topical use.

Embodiment 29 The cosmetic or dermatologic or pharmaceutical composition of embodiment 28, wherein the effective amount of the compound for use in regenerating the sensory neuron neurite length in an ageing human cell is in the range of from 0.01 to 10 weight-%, preferably in the range of from 0.025 to 5 weight-%, more preferably in the range of from 0.05 to 1.5 weight-%, more preferably in the range of from 0.1 to 1.0 weight-%, based on the weight of the cosmetic or dermatologic or pharmaceutical composition.

Embodiment 30 The cosmetic or dermatologic or pharmaceutical composition of embodiment 28 or 29, being in the form of a cream, a serum, an oil-in-water emulsion, a water-in-oil emulsion, an oil, a gel, an aqueous solution, a vectorization system, an anhydrous stick, a foam, a lotion, a milk, or a camouflage product, preferably in the form of a cream or a serum.

Embodiment 31 The cosmetic or dermatologic or pharmaceutical composition of any one of embodiments 26 to 30, further comprising a cosmetic or dermatologic or pharmaceutical acceptable carrier, preferably the acceptable carrier is selected from the group consisting of water, glycerin, glycol, propanediol, and mixtures of two or more thereof, more preferably selected from the group consisting of water, glycerin, and mixtures of two or more thereof.

Embodiment 32 The cosmetic or dermatologic or pharmaceutical composition of any one of embodiments 26 to 31, further comprising one or more emulsifiers.

Embodiment 33 The cosmetic or dermatologic or pharmaceutical composition of embodiment 32, wherein the one or more emulsifiers are selected from the group consisting of a mixture of cetearyl olivate and sorbitan olivate; xanthan gum; ceteareth-6 olivate; cetearyl alcohol; ceteth-20; glyceryl stearate; steareth-20; a mixture of cetyl alcohol, glyceryl stearate, PEG-75 stearate, ceteth-20 and stearaeth-20; carbomer; a mixture of sodium acrylate and sodium acryloyldimethyl taurate copolymer; a mixture of beheneth-30 phosphate, cetostearyl alcohol, dicetyl phosphate and cetyl phosphate; and mixtures of two or more thereof, preferably selected from the group consisting of a mixture of cetearyl olivate and sorbitan olivate; xanthan gum; ceteareth-6 olivate; and mixtures of two or more thereof.

Embodiment 34 The cosmetic or dermatologic or pharmaceutical composition of any one of embodiments 26 to 33, further comprising one or more emollients.

Embodiment 35 The cosmetic or dermatologic or pharmaceutical composition of embodiment 34, wherein the one or more emollients are selected from the group consisting of isocetyl stearate; dicaprylyl carbonate; octyldodecanol; stearyl dimethicone; neopentyl glycol diethylhexanoate; cetyl ricinoleate; shorea robusta seed butter; dicaprylyl ether; cyclomethicone; a mixture of isodecane, hydrogenated tetradecenyl and methylpentadecene; and mixtures of two or more thereof, preferably selected from the group consisting of isocetyl stearate; dicaprylyl carbonate; octyldodecanol; stearyl dimethicone; neopentyl glycol diethylhexanoate; cetyl ricinoleate; shorea robusta seed butter; and mixtures of two or more thereof.

Embodiment 36 The cosmetic or dermatologic or pharmaceutical composition of any one of embodiments 26 to 35, further comprising one or more of a pH regulator, a preservative, a thickening agent, a skin-care agent, and a perfuming agent.

Embodiment 37 The cosmetic or dermatologic or pharmaceutical composition of embodiment 26 or 27, being for oral use.

Embodiment 38 The cosmetic or dermatologic or pharmaceutical composition of embodiment 37, wherein the effective amount of the compound for use in regenerating the sensory neuron neurite length in an ageing human cell is in the range of from 1 to 100 weight-%, more preferably in the range of from 1 to 95 weight-%, preferably in the range of from 2 to 70 weight-%, more preferably in the range of from 4 to 55 weight-%, based on the weight of the cosmetic or dermatologic or pharmaceutical composition.

Embodiment 39 The cosmetic or dermatologic or pharmaceutical composition of embodiment 37 or 38, being in the form of a liquid, a capsule, a gummy, a powder, or a gel.

Embodiment 40 The cosmetic or dermatologic or pharmaceutical composition of any one of embodiments 37 to 39, further comprising a antioxidant and/or anti-inflammatory component, preferably said component being selected from the group consisting of zinc, OPCs, OPC derivatives, lutein, glutathione, selenium, vitamin C, blueberries, pomegranate, zinc bisglycinate, matcha tea, vitamin A, vitamin E, N-acetyl-cysteine, zeaxanthin, lycopene, turmeric, astaxanthin, and mixtures of two or more thereof, more preferably selected from the group consisting of zinc, OPCs, OPC derivatives, lutein, glutathione, selenium, vitamin C, blueberries, and mixtures of two or more thereof.

Embodiment 41 The cosmetic or dermatologic or pharmaceutical composition of any one of embodiments 37 to 40, further comprising one or more co-active ingredients, preferably one or more of hyaluronic acid, collagen, superoxide dismutase (SOD), vitamins, polyphenols, peptides, saccharides, and lipides.

Embodiment 42 The cosmetic or dermatologic or pharmaceutical composition of any one of embodiments 37 to 41, further comprising a flavoring ingredient.

Embodiment 43 The cosmetic or dermatologic or pharmaceutical composition of any one

embodiments 37 to 42, wherein the daily dosage of the compound for use in regenerating the sensory neuron neurite length in an ageing human cell *(active ingredient)* is in the range of from 10 mg/day to 1g/day, preferably in the range of from 50 mg/day to 750 mg/day, more preferably in the range of from 100 mg/day to 500 mg/day.

Embodiment 44 A non-therapeutical method for regenerating the sensory neuron neurite length in an ageing human cell, comprising
applying a cosmetic or dermatologic or pharmaceutical composition for topical use according to any one of embodiments 26 to 36 to skin in need at least once a day, preferably twice a day; or
administering a cosmetic or dermatologic or pharmaceutical composition for oral use according to any one of embodiments 26, 27 and 37 to 43 to a person in need once or twice a day.

Embodiment 45 Sialic acid and/or any derivatives thereof for use in regenerating the sensory neuron neurite length in an ageing human cell, preferably an ageing human epidermal cell.

Embodiment 46 Use of sialic acid as the active ingredient in a cosmetic or dermatologic composition for anti-ageing and/or skin regeneration.

Embodiment 47 Use of sialic acid and/or any derivatives thereof for enhancing or promoting the synthesis of one or more of oxytocin, serotonin and beta-endorphin, preferably by epidermal cells and/or neurons, more preferably by keratinocytes and /or neurons.

In the context of the present invention, the term "human ageing cell" refers to a cell of an ageing human being, for example a human of over 25 years-old or preferably over 30 years old and more.

In the context of the present invention, the term "one or more of A, B and C" in an expression disclosing several possible features/possibilities, wherein each of A, B and C stands for specific features/possibilities, is to be understood as disclosing that explicitly either A, or B, or C, or A and B, or A and C, or B and C, or A and B and C. In this regard, it is noted that the skilled person is capable of transfer to the above abstract term to a concrete example. For example, in the context of the present invention, when it is disclosed "for enhancing or promoting the synthesis of one or more of oxytocin, serotonin and beta-endorphin", it disclosed that a given compound/composition can enhance or promote the synthesis of oxytocin, or serotonin, or beta-endorphin, or oxytocin and serotonin, or serotonin and beta-endorphin, or oxytocin and beta-endorphin, or oxytocin, serotonin and beta-endorphin.

The present invention is further illustrated by the following examples.

### EXAMPLES

### I. Oxytocin, Serotonin and Beta-endorphin synthesis - Neuromodulation (well-being, pleasure, serenity)

### I.1 Material and Methods

### 1.1 Tested compound

The compound "Sialic Acid" ("inventive product") was provided by SHANDONG TIANSHENG BIOTECHNOLOGY CO.

### 1.2. Assay system

The keratinocytes used herein were human epidermal keratinocyte cells were obtained from a 49-year-old donor. For performing the experiments, keratinocytes have been cultivated in monolayer until reaching confluency in keratinocyte culture medium at 37°C and 5% CO₂.

### 1.3. Incubation protocol

Cells were pre-incubated during 24 hours in absence (control) or in presence of the tested compound at different concentration for sialic acid, namely it has been tested at 0.02; 0.06 and 0.2% (w/v)) in water.

### 1.4. Sialic acid compounds preparation

The tested compound named Sialic Acid was solubilized directly in the incubation medium (keratinocyte culture medium) at 2% (w/v). The solution obtained was diluted directly in the incubation medium in order to reach the different concentrations described above.

### 1.5. Evaluation of the effects

### 1.5. 1 Measurement of oxytocin synthesis

At the end of the 24 hours' incubation period, oxytocin released in the incubation medium was quantified using a sensitive and specific ELISA kit.

### 1.5. 2 Measurement of serotonin synthesis

At the end of the 24 hours' incubation period, serotonin released in the incubation medium was quantified using a sensitive and specific ELISA kit.

### 1.5. 3 Measurement of beta-endorphin synthesis

At the end of the 24 hours' incubation period, beta-endorphin released in the incubation medium was quantified using a sensitive and specific ELISA kit.

### 1.5. 4 Protein quantification

At the end of the incubation period, proteins contained in the cell lysates have been quantified using a spectro-colorimetric method, namely the Bradford method.

### 1.6. Statistics

Results are expressed in
pg of oxytocin per mg of proteins (Mean ± S.D.).
ng of serotonin per mg of proteins (Mean ± S.D.).
pg of beta-endorphin per mg of proteins (Mean + S.D.).

Level of significance between "Control" and "test compound" has been assessed independently for each product by a one factor variance analysis (One way ANOVA) followed by a Holm-Sidak test (*: p<0.05).

### I.2 Effect of sialic acid compounds on oxytocin, serotonin and beta-endorphin synthesis in keratinocytes

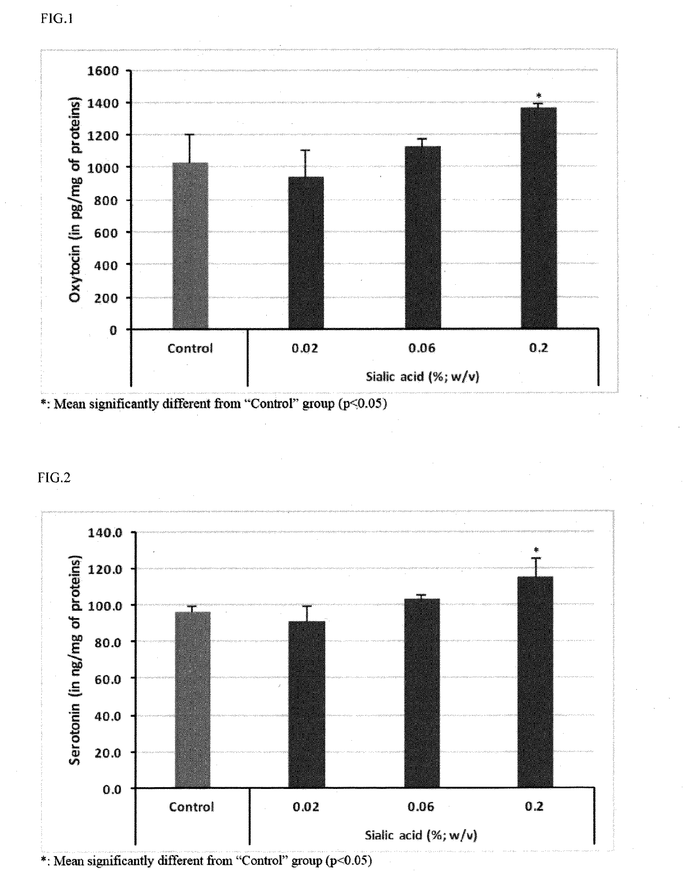

As may be taken from FIG. 1, the sialic acid compound tested at 0.02 % (w/v) didn't show any increase of oxytocin production compared to the "control". However, the sialic acid compound tested at 0.06% (w/v) shows an improvement in **oxytocin** synthesis in comparison with "control". Tested at 0.2% (w/v), the sialic acid compound significantly increased oxytocin synthesis by **+32.5%** (p<0.05) in comparison with "control". Thus, it has been demonstrated that sialic acid compounds permit an increase in oxytocin synthesis and/or oxytocin secretion by keratinocytes, and this results in the neuromodulation by restoring sensorial factors and by providing well-being feeling.

As may be taken from FIG.2, the sialic acid compound tested at 0.02 % (w/v) didn't show any increase of serotonin production compared to the "control". However, the sialic acid compound tested at 0.06% (w/v) shows an improvement in **serotonin** synthesis in comparison with "control". Tested at 0.2% (w/v), the sialic acid compound significantly increased serotonin synthesis by **+19.6%** (p<0.05) in comparison with "Control". Thus, it has been demonstrated that sialic acid compounds permit an increase in serotonin synthesis and/or serotonin secretion by keratinocytes, and this results in the positive neuromodulation of mood and reducing pain.

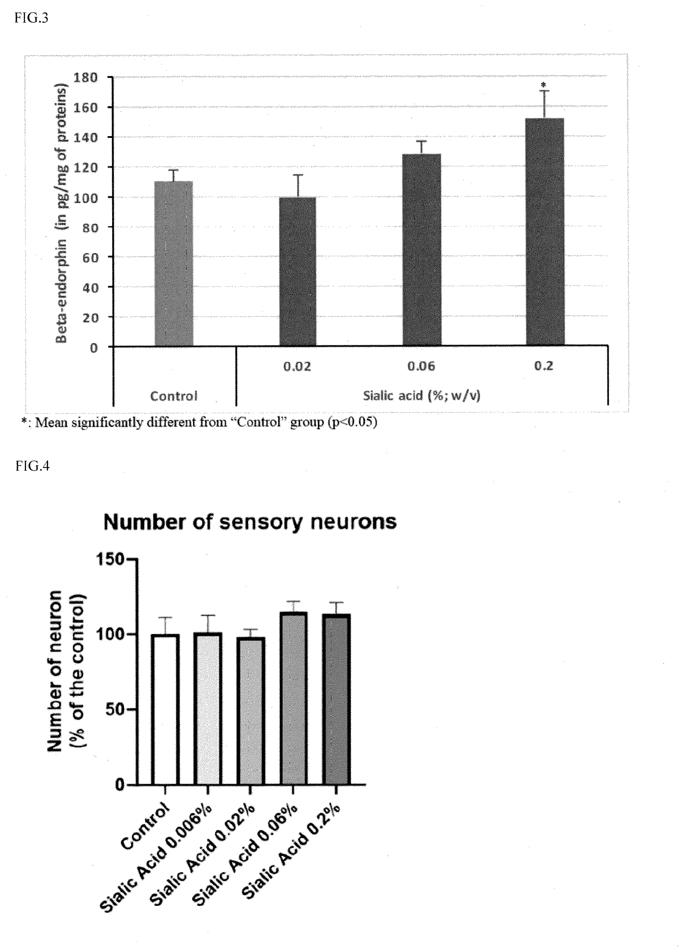

As may be taken from FIG. 3, the sialic acid compound tested at 0.02 % (w/v) didn't show any increase of serotonin production compared to the "control". However, the sialic acid compound tested at 0.06% (w/v) shows an improvement in **beta-endorphin** synthesis in comparison with "control". Tested at 0.2% (w/v), the sialic acid compound significantly increased beta-endorphin synthesis by **+38.1%** (p<0.05) in comparison with "control". Thus, it has been demonstrated that sialic acid compounds permit an increase in beta-endorphin synthesis and/or beta-endorphin secretion by keratinocytes, and this results in a neuromodulation to reduce stress and provide well-being feeling.

### II. Sensory Neuron Neurite Length Regeneration - Neuroprotection/Anti-ageing

### II.1. Experimental protocol

### 1.1. Culture and differentiation of human sensory neurons and co-culture with keratinocytes

The sensory neurons are derived from hiPS cells (human induced Pluripotent Stem cells). The cells were seeded and maintained in culture inducing their differentiation (M1) at 37°C and 5% CO₂. After differentiation, the M1 medium was changed to coculture medium (M2). The keratinocytes were seeded in the plates above the layer of differentiated hiPS. The cells were maintained in culture at 37°C and 5% CO₂.

### 1.2. Evaluation of the effect of compounds on the neurite outgrowth of human sensory neurons in co-culture with keratinocytes

After validation of the absence of cytotoxicity of sialic acid at 0.2%, 0.06%, 0.002% (w/v), cultures were done according to the conditions described in paragraph 1.1 in the presence of keratinocytes from an elderly donor (57 years old) or a young donor (23 years old). The following conditions were done:
- Control medium (keratinocytes from a 23 y/o donor)
- Control medium + Neuron Growth Factor (NGF) 50ng/mL (keratinocytes from a 23 y/o donor)
- Control medium + Sialic Acid at 0.2%, 0.06% and 0.02% (w/v) (keratinocytes from a 23 y/o donor)
- Control medium (keratinocytes from a 57 y/o donor)
- Control medium + NGF 50ng/mL (keratinocytes from a 57 y/o donor)
- Control medium + Sialic Acid at 0.2%, 0.06% and 0.02% (w/v) (keratinocytes from a 57 y/o donor) Per culture condition, 6 wells were done. After 4 days of incubation in the presence of the active ingredients and the cells were fixed in a 4% PFA solution. The cultures were then washed twice in PBS then permeabilized and the non-specific sites were blocked. The cells were incubated in the presence of an anti-β-tubulin primary antibody. This antibody was revealed by a secondary antibody coupled to a fluorochrome. The nuclei were labeled with Hoechst's solution, a nuclear fluorescent marker.

### II.2 Effect of sialic acid on the sensory neuron neurite length regeneration

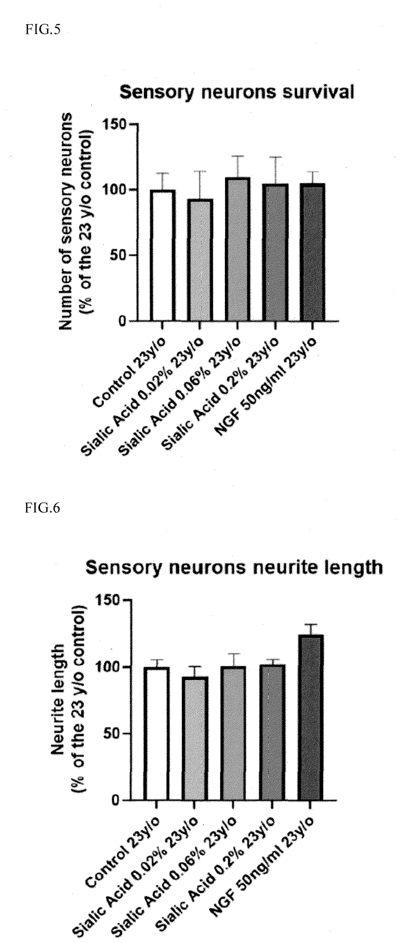

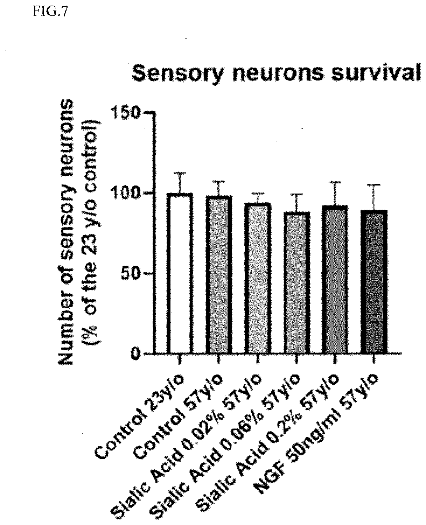

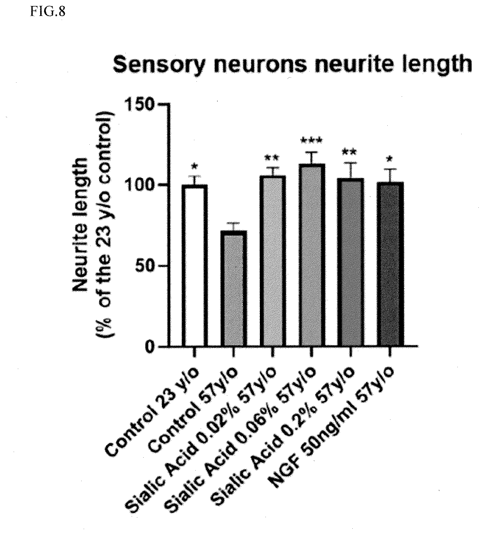

FIG. 4 shows that with sialic acid at the tested concentrations did not decrease the number of sensory neurons showing that no cellular mortality was induced by this compound (no cytotoxicity). FIG. 5 shows that there is no particular effect of sialic acid on a young person (23 y/o) as there is the same number of neurons with or without the sialic acid compound. FIG.6 shows as FIG. 5 that there is no particular effect of sialic acid on a young person as the length of the sensory neurons neurite is maintained with or without sialic acid compounds. FIG.7 shows that, in terms of survival of the sensory neurons, sialic acid compounds have no particular effect for the 23 y/o and 57 y/o donors. However, it is evident from FIG.8 that sialic acid compounds at the tested concentrations permit to regenerate the length of the sensory neurons neurite of the 57 y/o donor such that the length after treatment is similar to or higher than the length of the 23 y/o donor. Thus, it has been demonstrated that sialic acid compounds permit the regeneration of sensory neurons neurite length in co-culture of neurons and keratinocytes, and this results in the regeneration of cells, such as neurons neurite length, and permits to obtain a young sensory neuron phenotype. It has been demonstrated that sialic acid compounds play an anti-ageing role as well as a neuroprotection role.

### III. Formulations

### III.1 Cosmetics formulations (topical use)

### 1.1 Serum well being

| INCI Name - Ingredients | Wt.-% |
|---|---|
| ***PHASE A*** | |
| Aqua | q.s. 100 |
| Phenoxyethanol | 0.90 |
| Glycerin | 4.00 |
| Xanthan gum | 0.20 |
| Acetylneuraminic acid | 0.5 |
| Sodium hydroxide | q.s. (pH 5.5 - 6.0) |

| ***PHASE B*** | |
|---|---|
| Cetearyl Olivate (and) Sorbitan Olivate | 2.50 |
| Isocetyl stearate | 2.5 |
| Tetradecane | 2.5 |

| | |
|---|---|
| Protocol: Prepare Phase A by mixing the ingredients at 75°C. Prepare Phase B by mixing the ingredients at 75°C. Create an emulsion by pouring B into A with vigorous agitation. Readjust pH with sodium hydroxide. | |

### 1.2 Cream for Neuro-regeneration

| INCI Name - Ingredients | Wt.-% |
|---|---|
| ***PHASE A*** | |
| Cetearyl olivate / Sorbitan olivate | 5.00 |
| Ceteareth-6 olivate | 2.00 |
| Octyldodecanol | 2.00 |
| Dicaprylyl carbonate | 4.00 |
| Caprylyl methicone | 3.00 |
| Stearyl dimethicone | 3.00 |
| Neopentyl glycol diethylhexanoate | 3.00 |
| Cetyl ricinoleate | 5.00 |
| Shorea robusta seed butter | 5.00 |

| ***PHASE B*** | |
|---|---|
| Aqua | q.s. 100 |
| Phenoxyethanol | 0.9 |
| Acrylates/C10-30 alkyl acrylate crosspolymer | 0.15 |

| ***PHASE C*** | |
|---|---|
| Acetylneuraminic acid | 0.20 |
| | |
| Sodium hydroxide | q.s. pH 4.7 |

| | |
|---|---|
| Protocol: Prepare Phase A by mixing the ingredients at 70°C. Prepare Phase B by mixing the ingredients at 70°C. Create an emulsion by pouring A into B with vigorous stirring. Cool while stirring. At 45°C, add the elements of phase C, and then adjust the pH with sodium hydroxide. | |

### III.2 Dietary supplement formulae

| **Formula A** | **Anti-aging/Neuroregeneration** | |
|---|---|---|
| 3g per stick | | |
| | Dose/stick | Daily dose |
| Sialic acid | 300 mg | 300 mg |
| Full spectrum hyaluronic acid | 180 mg | 180 mg |
| Marine collagen | 500 mg | 500 mg |
| Liposomal Vitamin C | 100 mg | 100 mg |
| Blueberries powder qsp 3g | 1900 mg | 1900 mg |
| Blueberries flavoring | 20 mg | 20 mg |

| **Formula B** | **Neuroprotection skin formula** | Daily dose |
|---|---|---|
| Capsule 300 mg | | 150 mg |
| Glutathione | 100 mg | 100 mg |
| Superoxide dismutase (SOD) | 20 mg | 20 mg |
| Selenium | 0.055 mg | 0.055 mg |
| Excipients | q.s. (300 mg) | |

| **Formula C** | **Serenity/Neuromodulation** | |
|---|---|---|
| Gummy of 1g | | |
| Dosage: 2 gummies/jour | | |
| | Dose/Gummy | Daily dose |
| Sialic acid | 50 mg | 100 mg |
| Zinc | 5 mg | 10 mg |
| Lutein | 3 mg | 6 mg |
| Grapes OPC* | 50 mg | 100 mg |
| Excipients | q.s. (1g) | |

| | | |
|---|---|---|
| * oligoproanthocyanidins | | |

### Description of the figures

FIG.1 represents an evaluation of the effect of the sialic acid compounds on oxytocin synthesis by human normal keratinocytes.
FIG.2 represents an evaluation of the effect of the sialic acid compounds on serotonin synthesis by human normal keratinocytes in monolayer culture.
FIG.3 represents an evaluation of the effect of the sialic acid compounds on beta-endorphin synthesis by human normal keratinocytes.
FIG.4 represents an evaluation of the effect of sialic acid compounds on the survival of sensory neurons. Co-culture of neurons & keratinocytes was incubated for 4 days in presence or not of the test compound. The survival of sensory neurons was determined by immunostaining assay using an anti-β-tubulin antibody and data has been expressed in percentage of the "control" condition (= "Non treated control"; 100%). Mean +/- s.e.m. of 6 replicates by treatment conditions. Statistics were done using a Student test with the "control" condition as reference.
FIG.5 represents an evaluation of the effect of sialic acid compounds on the survival of sensory neurons in the model with keratinocytes from a 23y/o donor. Co-culture was incubated for 4 days in presence or not of the test compound or the positive control NGF. The survival of sensory neurons was determined by immunostaining assay using an anti-β-tubulin antibody and data has been expressed in percentage of the "control 23y/o" condition (= "Non treated control"; 100%). Mean +/- s.e.m. of 6 replicates by treatment conditions. Statistics were done using a One Way ANOVA test followed by Dunnett correction with the "control 23y/o" condition as reference.
FIG.6 represents an evaluation of the effect of sialic acid compounds on neurite length of sensory neurons in the model with keratinocytes from a 23-year-old donor. Co-culture was incubated for 4 days in presence or not of the test compound or the positive control NGF. The neurite length was determined by immunostaining assay using an anti-β-tubulin antibody. Data has been normalized by the number of sensory neurons on the corresponding well and expressed in percentage of the "control 23y/o" condition (= "Non treated control"; 100%). Mean +/- s.e.m. of 6 replicates by treatment conditions. Statistics were done using a One Way ANOVA test followed by Dunnett correction with the "control 23y/o" condition as reference.
FIG.7 represents an evaluation of the effect of sialic acid compounds on the survival of sensory neurons in the model with keratinocytes from a 57y/o donor. Co-culture was incubated for 4 days in presence or not of the test compound or the positive control NGF. The survival of sensory neurons was determined by immunostaining assay using an anti-β-tubulin antibody and data has been expressed in percentage of the "control 23y/o" condition (= "Non treated control"; 100%). Mean +/- s.e.m. of 6 replicates by treatment conditions. Statistics were done using a One Way ANOVA test followed by Dunnett correction with the "control 57y/o" condition as reference.
FIG.8 represents an evaluation of the effect of sialic acid compounds on neurite length of sensory neurons in the model with keratinocytes from a 57-year-old donor. Co-culture was incubated for 4 days in presence or not of the test compound or the positive control NGF. The neurite length was determined by immunostaining assay using an anti-β-tubulin antibody. Data has been normalized by the number of sensory neurons on the corresponding well and expressed in percentage of the "control 23y/o" condition (= "Non treated control"; 100%). Mean +/- s.e.m. of 6 replicates by treatment conditions. Statistics were done using a One Way ANOVA test followed by Dunnett correction with the "control 57y/o" condition as reference (* p<0.05; ** p<0.01; *** p<0.001).

## Claims

1. Use of sialic acid and/or any derivatives thereof as an effective ingredient in a neuro-cosmetic or neuro-dermatologic composition for enhancing or promoting the synthesis of one or more of oxytocin, serotonin and beta-endorphin and/or for regenerating the sensory neuron neurite length in an ageing human cell.

2. The use of claim 1, for enhancing or promoting the synthesis of one or more of oxytocin, serotonin and beta-endorphin.

3. The use of claim 1, for regenerating the sensory neuron neurite length in an ageing human cell.

4. Cosmetic or dermatologic or pharmaceutical composition comprising
an effective amount of a compound for use in enhancing or promoting the synthesis of one or more of oxytocin, serotonin and beta-endorphin;
wherein the compound for use in enhancing or promoting the synthesis of one or more of oxytocin, serotonin and beta-endorphin is sialic acid and/or any derivatives thereof.

5. The cosmetic or dermatologic or pharmaceutical composition of claim 4, wherein the compound for use in enhancing or promoting the synthesis of one or more of oxytocin, serotonin and beta-endorphin is sialic acid.

6. The cosmetic or dermatologic or pharmaceutical composition of claim 4 or 5, being for topical use, preferably the composition is in the form of a cream, a serum, an oil-in-water emulsion, a water-in-oil emulsion, an oil, a gel, an aqueous solution, a vectorization system, an anhydrous stick, a foam, a lotion, a milk, or a camouflage product, more preferably in the form of a cream or a serum.

7. The cosmetic or dermatologic or pharmaceutical composition of claim 6, wherein the effective amount of the compound for use in enhancing or promoting the synthesis of one or more of oxytocin, serotonin and beta-endorphin is in the range of from 0.01 to 10 weight-%, preferably in the range of from 0.025 to 5 weight-%, more preferably in the range of from 0.05 to 1.5 weight-%, more preferably in the range of from 0.1 to 1.0 weight-%, based on the weight of the cosmetic or dermatologic or pharmaceutical composition.

8. The cosmetic or dermatologic or pharmaceutical composition of any one of claims 4 to 7, further comprising one or more emulsifiers; wherein the one or more emulsifiers are selected from the group consisting of a mixture of cetearyl olivate and sorbitan olivate; xanthan gum; ceteareth-6 olivate; cetearyl alcohol; ceteth-20; glyceryl stearate; steareth-20; a mixture of cetyl alcohol, glyceryl stearate, PEG-75 stearate, ceteth-20 and stearaeth-20; carbomer; a mixture of sodium acrylate and sodium acryloyldimethyl taurate copolymer; a mixture of beheneth-30 phosphate, cetostearyl alcohol, dicetyl phosphate and cetyl phosphate; and mixtures of two or more thereof; preferably selected from the group consisting of a mixture of cetearyl olivate and sorbitan olivate; xanthan gum; ceteareth-6 olivate; and mixtures of two or more thereof.

9. The cosmetic or dermatologic or pharmaceutical composition of any one of claims 4 to 8, further comprising one or more emollients; wherein the one or more emollients are selected from the group consisting of isocetyl stearate; dicaprylyl carbonate; octyldodecanol; stearyl dimethicone; neopentyl glycol diethylhexanoate; cetyl ricinoleate; shorea robusta seed butter; dicaprylyl ether; cyclomethicone; a mixture of isodecane, hydrogenated tetradecenyl and methylpentadecene; pentaerythrityl tetralaurate; and mixtures of two or more thereof; preferably selected from the group consisting of isocetyl stearate; dicaprylyl carbonate; octyldodecanol; stearyl dimethicone; neopentyl glycol diethylhexanoate; cetyl ricinoleate; shorea robusta seed butter; and mixtures of two or more thereof.

10. The cosmetic or dermatologic or pharmaceutical composition of claim 4 or 5, being for oral use; preferably the composition is in the form of a liquid, a capsule, a gummy, a powder, or a gel.

11. The cosmetic or dermatologic or pharmaceutical composition of claim 10, wherein the effective amount of the compound for use in enhancing or promoting the synthesis of one or more of oxytocin, serotonin and beta-endorphin is in the range of from 1 to 100 weight-%, preferably in the range of from 2 to 70 weight-%, more preferably in the range of from 4 to 55 weight-%, based on the weight of the cosmetic or dermatologic or pharmaceutical composition.

12. The cosmetic or dermatologic or pharmaceutical composition of claim 10 or 11, further comprising an antioxidant and/or anti-inflammatory component, preferably said component being selected from the group consisting of zinc, OPCs, OPC derivatives, lutein, glutathione, selenium, vitamin C, blueberries, pomegranate, zinc bisglycinate, matcha tea, vitamin A, vitamin E, N-acetyl-cysteine, zeaxanthin, lycopene, turmeric, astaxanthin, and mixtures of two or more thereof, more preferably selected from the group consisting of zinc, OPCs, OPC derivatives, lutein, glutathione, selenium, vitamin C, blueberries, and mixtures of two or more thereof.

13. The cosmetic or dermatologic or pharmaceutical composition of any one of claims 10 to 12, wherein the daily dosage of the compound for use in enhancing or promoting the synthesis of one or more of oxytocin, serotonin and beta-endorphin is in the range of from 10 mg/day to 1 g/day, preferably in the range of from 50 mg/day to 750 mg/day, more preferably in the range of from 100 mg/day to 500 mg/day.

14. A non-therapeutical method for increasing the synthesis of one or more of oxytocin, serotonin and beta-endorphin, preferably by epidermal cells and/or neurons, more preferably by keratinocytes and/or neurons, in order to induce a feeling of well-being, pleasure, and/or serenity in a human person, comprising
applying a cosmetic or dermatologic or pharmaceutical composition for topical use according to any one of claims 4 to 8 to skin in need at least once a day; or
administering a cosmetic or dermatologic or pharmaceutical composition for oral use according to any one of claims 4, 5 and 10 to 13 to a person in need once or twice a day.

15. Cosmetic or dermatologic or pharmaceutical composition comprising
an effective amount of a compound for use in regenerating the sensory neuron neurite length in an ageing human cell, and
wherein the compound for use in regenerating the sensory neuron neurite length in an ageing human cell is sialic acid and/or any derivatives thereof.

16. The cosmetic or dermatologic or pharmaceutical composition of claim 15, being for topical use; wherein the effective amount of the compound for use in regenerating the sensory neuron neurite length in an ageing human cell is in the range of from 0.01 to 10 weight-%, preferably in the range of from 0.025 to 5 weight-%, more preferably in the range of from 0.05 to 1.5 weight-%, more preferably in the range of from 0.1 to 1.0 weight-%, based on the weight of the cosmetic or dermatologic or pharmaceutical composition; or
being for oral use; wherein the effective amount of the compound for use in regenerating the sensory neuron neurite length in an ageing human cell is in the range of from 1 to 100 weight-%, preferably in the range of from 2 to 70 weight-%, more preferably in the range of from 4 to 55 weight-%, based on the weight of the cosmetic or dermatologic or pharmaceutical composition.

17. A non-therapeutical method for regenerating the sensory neuron neurite length in an ageing human cell, comprising
applying a cosmetic or dermatologic or pharmaceutical composition for topical use according to claim 15 or 16 to skin in need at least once a day; or
administering a cosmetic or dermatologic or pharmaceutical composition for oral use according to claim 15 or 16 to a person in need once or twice a day.
